Europäisches Patentamt

European Patent Office

Office européen des brevets

(11) Publication number: **0 184 928**
**B1**

(12) **EUROPEAN PATENT SPECIFICATION**

(45) Date of publication of patent specification: **16.08.90**

(51) Int. Cl.⁵: **A 61 N 5/06, A 61 H 39/00**

(21) Application number: **85308874.8**

(22) Date of filing: **05.12.85**

(54) Hygienic attachments for therapy lasers.

(30) Priority: **06.12.84 AU 8453/84**

(43) Date of publication of application:
**18.06.86 Bulletin 86/25**

(45) Publication of the grant of the patent:
**16.08.90 Bulletin 90/33**

(84) Designated Contracting States:
**AT BE CH DE FR GB IT LI LU NL SE**

(56) References cited:
**EP-A-0 054 296**
**FR-A-2 202 674**
**FR-A-2 329 258**
**FR-A-2 420 352**
**FR-A-2 513 109**
**GB-A-1 537 696**
**GB-A-2 071 500**

(73) Proprietor: **HUGHES TECHNOLOGY PTY LTD.**
**P.O. Box 1155**
**Canberra City, ACT 2601 (AU)**

(72) Inventor: **Hughes, John Leonard**
**32 Basedow Street**
**Torrens 2607 (AU)**
Inventor: **Irons, Frank Edward**
**13 Beach Place**
**Holt 2615 (AU)**
Inventor: **Kwiatkowski, George**
**36 Maharatta Circuit**
**Isabella Plains 2905 (AU)**

(74) Representative: **Hartley, David et al**
**Withers & Rogers 4 Dyer's Buildings Holborn**
**London, EC1N 2JT (GB)**

Courier Press, Leamington Spa, England.

**Description**

The invention relates to a hand-held laser therapy laser beam generating system and more particularly a system for ensuring hygienic contact between a laser therapy unit, the therapist administering the laser beam treatment and the patient receiving said treatment.

The advent of the laser in 1960 heralded a new era in the ancient art of acupuncture treatment, which had previously been solely based on the use of needles to stimulate acupuncture points on human and animal bodies.

In 1966, one of us (John Leonard Hughes) whilst working at the Defence Research Establishment in South Australia focused a three watts output of a blue-green argon ion laser onto his hand and experienced a needle-like sensation.

Others used much lower powered lasers, in particular helium-neon lasers, emitting only a few milli-watts in the red, and claimed that acupuncture points could be stimulated at such low power levels without needle-like sensations.

Using acupuncture needles, the therapist inserts them into the patients through points reputed to affect the body in beneficial ways. When such needles are inserted into the patient's body, they can be mechanically vibrated to increase the claimed beneficial effects. Similarly, with laser beam stimulation of acupuncture points, the light beam can be modulated in intensity with claimed beneficial effects by laser therapists worldwide. As with acupuncture needles, treatments with laser beams can include pain relief, with the added advantage of deep wound treatments. However, the unique advantage of laser beam therapy over acupuncture needle therapy is the fact that it is impossible to contaminate the laser beam as is the case with acupuncture needles.

Prior art laser therapy units are of two kinds, those with direct beam outputs and tho whose outputs are delivered to the patient via a flexible optical fibre bundle. A fundamental problem with prior art laser therapy units is the fact that the output heads which are held by the therapists and come into frequent contact with the patients particularly during internal body therapy, are difficult to sterilise. In the case of the direct beam laser therapy unit head, the whole head would have to be sealed to prevent the high voltage laser tube coming into contact with water. Similarly, the immersion of a fibre optical cable head would also require total head sealing to ensure full sterilising action. Such prior art laser therapy units are low cost devices operating under Class II international safety regulations with maximum output of one milliwatt continuous beam power. To provide a sealed, sterilisable head on such low cost units would cost more than the unit itself and would be uneconomical. Additionally, it would be difficult to provide 100% protection against transmissible diseases because such sealed laser therapy unit heads could not be made demountable in general use.

FR—A 2420352 discloses a hand-held laser therapy, laser beam generating system comprising a main body containing the laser beam generator and terminated by a conically-shaped output and which has a bore extending along the axis of the body, a hollow conically shaped detachable cap which fits over the conically shaped output end of the main body and is attachable to the head by means thereon engageable with means on the head.

It is an object of the present invention to avoid the disadvantages of prior art laser therapy systems.

In accordance with the present invention such a system is characterised in that the angular orientation of the cap is determined by protrusions in the cap sliding along guides in the head and in that the cap has a tube positioned along its inner, central axis and sealed to the tip of the cap and which is received by the bore so as to provide a laser output beam passage through the hollow tube and out of the cap.

The detachable end cap can be securely clipped onto the laser beam output tip of a therapy laser so as to prevent said laser output tip itself coming into contact with patients' bodies. If transmissible diseases such as acquired immune deficiency syndrome (AIDS) are picked up by the caps, then in the case where such caps are of metals such as stainless steel, they can be sterilised prior to further use, or to provide complete protection for both therapists and subsequent patients the caps can be of a plastic construction and simply disposed of after being used on a patient.

The invention makes it possible to ensure that the head of the laser therapy unit from which the laser beam emerges is also fully sterile as it comes into contact with either the patients' or therapists' bodies. It thus avoids or at least reduces the risk of spreading transmissible diseases during laser therapy treatments of humans and animals.

The disposable cap allows the therapist to treat areas which are difficult to access, such as the mouth, ear, and near the eyes which are sensitive to laser beam intensities.

Further, the cap may be adapted to convert a spot beam into a laser beam line which can then be spread into a rectangular or square distribution.

The laser beam output power from laser therapy units may be increased such that the intensity remains within the international Class II limits of one milliwatt per circular area of 7 millimetres diameter which is equivalent to the estimated area of a fully dilated pupil of the human eye.

In one embodiment, the detachable cap has embedded therein two electrodes which make contact with respective leads embedded in the laser unit head as the cap is fitted on the head so that the localised skin resistance between the two probe tips can be determined allowing for the laser beam output to be switched on to stimulate acupuncture points.

Embodiments of the invention will now be described by way of example with reference to the accompanying drawings in which:

Figure 1 is a schematic half cross-section of a hand-held laser therapy system head;

Figure 2 is a plan view of the half system head shown in Figure 1;

Figure 3 is a perspective view of a conical sterilisable and disposable cap for attachment to the head of Figures 1 and 2;

Figure 4 is a view similar to Figure 3 but of a cap fitted with skin resistance measuring electrodes;

Figure 5 is an enlarged view of part of the head shown in Figure 2 fitted with skin resistance measuring electrodes;

Figure 6 shows diagrammatically a hand-held laser therapy system incorporating a beam reflector focussing assembly;

Figure 7 shows diagrammatically the configuration of Figure 3 with a line forming extension which converts the cylindrically symmetrical laser output beam into a line beam of rectangular or square cross-section whose intensity per unit area satisfies International Class II safety standards but whose total power may exceed said standard in which case it is categorised International Class IIIA, the category of International Class IIIA corresponding to an expanded beam class II output; and

Figure 8 shows an embodiment for the 360 degree illumination of internal organs.

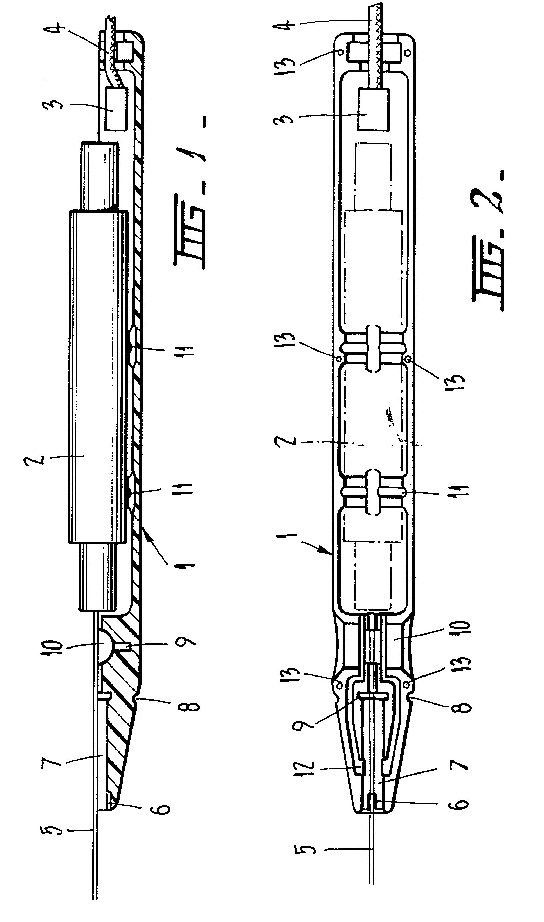

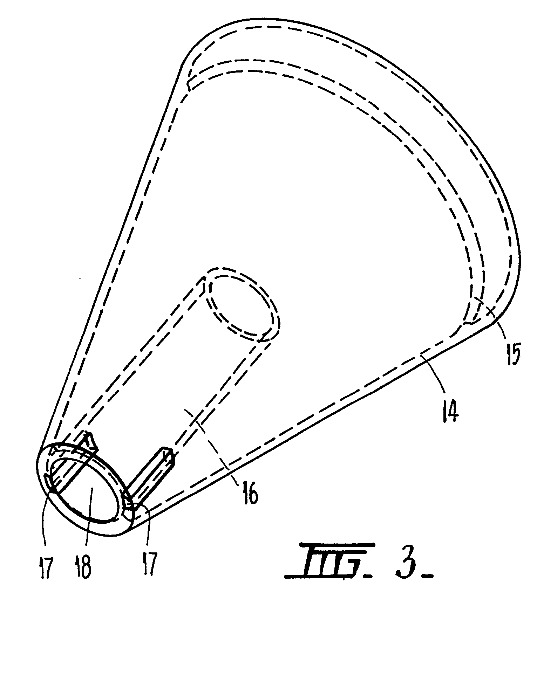

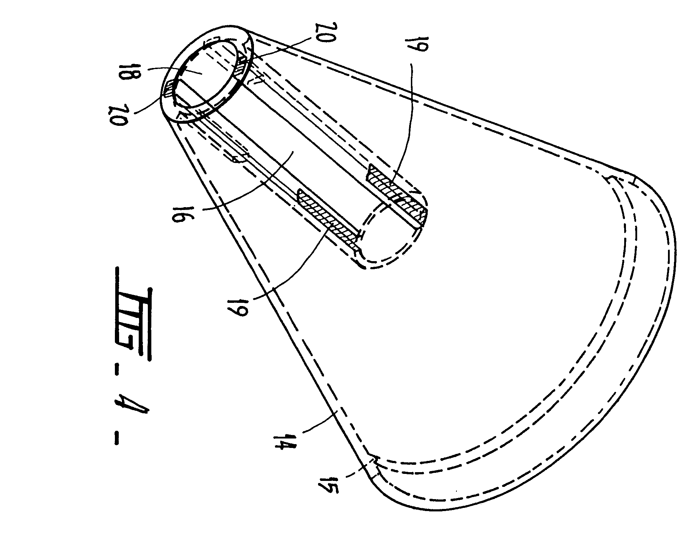

Figures 1 and 2 show a moulded plastic half-section 1 of a direct beam hand-held laser therapy unit head, containing a compact helium-neon laser beam-generating tube 2, mounted on two cradles 11 moulded into the section for receiving O-ring mountings around the tube 2, and the ballast resistors 3. A multicore, shielded electrical cable 4 connects the head assembly to the laser power supply at a control unit (not shown). The laser output beam 5 is indicated as it propagates through a channel 6 at the tip of the hand-held unit, formed when a similar second half-section (not shown) of the mould is attached by glue to the bottom half 1; the two half-sections being firmly clipped together at location points 13 prior to glueing. A locating groove 7 is provided for correctly orienting a detachable cap to be described below with reference to Figure 3 onto the laser therapy head whilst a groove 8 is used to firmly attach the cap onto the head. A groove 9 serves to locate either an optically polished window or an optically polished laser beam attenuator which also acts as a stop for glass containers filled with various liquids which can be inserted in the channel 6 and through which laser beam 5 can propagate. Recess 10 houses the safety switch necessary on Class II therapy lasers to block beam 5 as required.

A detachable cap intended to fit on the tip of the hand-held unit of Figures 1 and 2 is shown in Figure 2. It has a conical body 14 which fits over the end cone of body 1 and is formed with a circular rib 15 which engages in the groove 8 of the cone of body 1 so as to lock the cap into position during operation. The cap has a tube 16 positioned along its inner central axis and sealed to the tip of the cap. This fits into channel 6 of the Figure 1, to such a depth as to keep the possibility of contamination to an absolute minimum. Two locating ribs 17 orient the cap, via grooves 7 (Figure 1) onto the output head of body 1. In use, when the cap is attached to the laser therapy unit head the laser beam emerges through the output aperture 18. The cap may be of metal such as stainless steel, and thus sterilisable and re-usable or of plastic material permitting disposal after use.

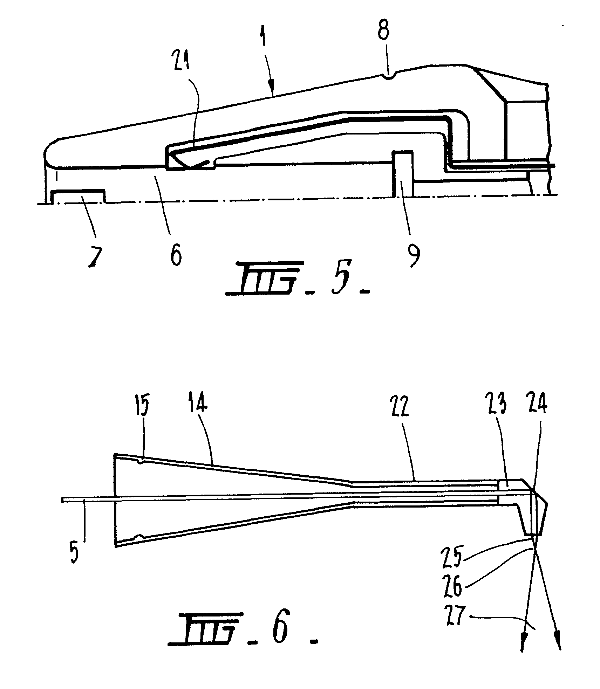

For the location of the acupuncture points, electrodes 19 used for the skin resistance measurements are fitted in the cap as shown in Figure 4, such that the tips 20 of the electrodes 19 are exposed for contact, in use with the body of the patient. For galvanic skin resistance measurements only one of the electrodes 19 is connected to the electrical circuits because the other contact is made via a hand-held electrode. However, for localised skin resistance measurements (LSR) both electrodes 19 are used with the skin between the two electrodes 20 forming part of the circuit.

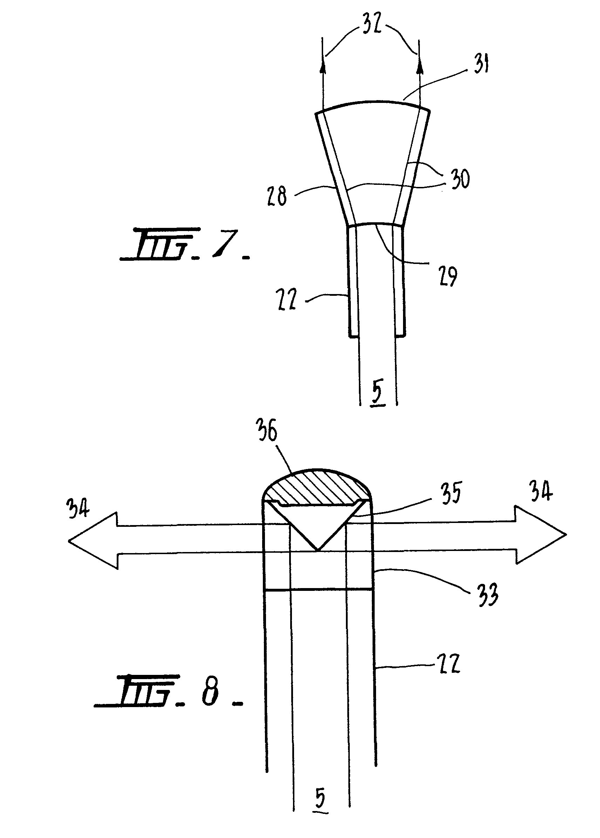

Figure 5, shows spring connectors 21 used to complete skin resistance measurement circuit when the cap shown in Figure 4 is attached to the laser therapy unit head 1.

The embodiment of Figure 6, includes a hollow extension stem 22 through which laser beam 5 propagates to the solid transparent head 23. The laser beam 5 is then totally internally reflected via the flat surface 24 onto the convex face 25 via an external focus spot 26 into the divergent beam 27. As far as eye safety is concerned focus points 26 will not be harmful to the eye for Class I safety lasers because the low power beam 5 expands into low intensity beam 27. Also low intensity beam 27 can be used to illuminate a larger area of the patient's body. On the other hand, fine focus point 26 is very useful for the treatment of delicate areas such as those on the ears or around the eyes where the standard laser beam output diameters are far too big for precision therapeutic treatments.

Figure 7 shows a solid optical element 28 through which laser beam 5 propagates after entering through the concave surface 29 and then expands via beam 30, to exit via the convex surface 31 to become the output beam 32 which can have a rectangular or square cross section.

In the embodiment of Figure 8, there is a transparent plastic cap 33 atom stem 22 through which beam 5 propagates to be reflected into 360 degree planar distribution 34 via reflection off a conically cut segment 35. The tip of the hand-held laser unit in his configuration is capped at 36.

**Claims**

1. A hand-held laser therapy, laser beam generating system comprising a main body (1), containing the laser beam generator (2) and terminated by a conically-shaped output end which has a bore (6) extending along the axis of the body, and a hollow, conically shaped detachable cap (14) which fits over the conically shaped output end of the main body (1) and is attached to the head by means (15) thereon engageable with means (8) on the head characterised in that the angular orientation of the cap is determined by protrusions (17) in the cap sliding along guides (7) in the head and in that the cap has a tube (16) positioned along its inner, central axis and sealed to the tip of the cap and which is received by the bore (6) so as to provide a laser output beam (5) passage through the hollow tube and out of the cap.

2. A system according to claim 1 wherein the cap (14) is firmly attachable to the head by a rib (15) on the inside surface of the cap which slips into a matching groove (8) in the head.

3. A system according to claim 2 wherein the detachable cap has embedded therein two electrodes (19) which make contact with respective leads (21) embedded in the laser unit head as the cap (14) is fitted on the head so that the localised skin resistance between the two probe tips (20) can be determined allowing for the laser beam output to be switched on to stimulate acupuncture points.

4. A system according to claim 2 wherein the detachable cap (14) has a hollow stem extension (22) through which the laser output beam (5) propagates, capped by a solid block (23) which is transparent to laser light and shaped in such a manner that the propagating laser beam is totally internally reflected through 90 degrees to exit from the cap via a convex surface which causes the emerging laser beam to focus into a fine spot in front of the convex surface (25), from which it diverges into a wide, conical beam (27) of decreasing intensity per unit area.

5. A system according to claim 4 wherein the hollow stem extension tube (2) is capped by a line-maker consisting of a transparent solid piece (28) whose input surface (29) is concave and whose output surface is convex (31) the emerging laser beam (32) being of rectangular or square cross-section providing for larger area illumination of the patients body.

6. A system according to claim 4 wherein the hollow stem (2) is capped by a transparent solid (33) whose end has a conical cavity (35) to provide the transmitted laser beam with total internal reflection into a 90 degree plane spread through 360 degrees for therapeutic treatments of internal organs in human and animal bodies.

7. A system according to claims 5 and 6 where the laser beam intensity per unit area is less than 1 milliwatt per circular area of 7 mm diameter but whose total laser beam output power exceeds one milliwatt for laser wavelengths across the electromagnetic spectrum.

**Patentansprüche**

1. Handgeführtes Lasertherapie-, laserstrahlerzeugendes System, das einen Hauptkörper (1) aufweist, der den Laserstrahlgenerator (2) enthält und von einem konisch geformten Austrittsende abgeschlossen ist, das eine entlang der Achse des Körpers verlaufende Bohrung (6) aufweist, mit einer hohlen, konisch geformten, abnehmbaren Kappe (14), die auf das konisch geformte Austrittsende des Hauptkörpers (1) paßt und am Kopf mit Hilfe von Mitteln (15) befestigt ist, die in Mittel (8) am Kopf eingreifen können, dadurch gekennzeichnet, daß die winklige Ausrichtung der Kappe durch Vorsprünge (17) in der Kappe bestimmt ist, die entlang führungen (7) in dem Kopf gleiten, und dadurch, daß die Kappe ein Rohr (16) aufweist, dasentlang ihrer inneren, zentralen Achse positioniert und dicht an der Spitze der Kappe befestigt ist und welches von der Bohrung (6) aufgenommen wird, um eine Passage für einen Laseraustrittsstrahl (5) durch das hohle Rohr und aus der Kappe heraus zu bilden.

2. System nach Anspruch 1, bei dem die Kappe (14) fest auf dem Kopf arretierbar ist durch eine Rippe (15) an der Innenfläche der Kappe, die in eine Paßnut (8) im Kopf einschnappt.

3. System nach Anspruch 2, bei dem in der abnehmbaren Kappe zwei Elektroden (19) eingebettet sind, die in Kontakt zu entsprechenden, im Lasereinheitskopf eingebetteten Leitungen (21) geraten, sobald die Kappe (14) auf den Kopf gesetzt ist, so daß der lokalisierte Hautwiderstand zwischen zwei Tastspitzen (20) bestimmt werden kann, um den Laserstrahlaustritt zum Stimulieren von Akupunkturpunkten anschalten zu können.

4. System nach Anspruch 2, bei dem die abnehmbare Kappe (14) einen hohlen Schaftauslauf (22) aufweist, durch den sich der Laseraustrittsstrahl (5) fortpflanzt, der durch einen massiven Block (23) abgedeckt ist, der für Laserlicht durchlässig ist und derart geformt ist, daß der sich ausbreitende Laserstrahl innen um 90° total reflektiert wird, um aus der Kappe über eine konvexe Fläche auszutreten, die den austretenden Laserstrahl dazu veranlaßt, sich in einem feinen Punkt vor der konvexen Fläche (25) zu fokussieren, von dem aus er in einen breiten konischen Strahl (27) von abnehmender Intensität pro Flächenbereich der Einheit divergiert.

5. System nach Anspruch 4, bei dem das hohle Schaftauslaufrohr (2) durch einen Richtungsgeber abgedeckt ist, der aus einem transparenten, massiven Stück (28) besteht, dessen Eintrittsfläche (29) konkav und dessen Austrittsfläche (31) konvex ist, wobei der austretende Laserstrahl (32) einen rechteckigen oder quadratischen Querschnitt besitzt, um einen vergrößerten Beleuchtungsbereich des Körpers des Patienten zu erlangen.

6. System nach Anspruch 4, bei dem der hohle Schaft (2) von einem transparenten Körper (33) abgedeckt ist, dessen Ende eine konische Vertiefung (35) besitzt, um den durchtretenden Laserstrahl mit einer inneren Totalreflektion in einer 90°-Ebene über 360° gestreut für therapeutische Behandlungen innerer Organe in menschlichen und tierischen Körpern zu versehen.

7. System nach den Ansprüchen 5 und 6, wobei die Laserstrahlintensität pro Einheitsfläche weniger als ein Milliwatt bei einer Kreisfläche von 7 mm Durchmesser beträgt, aber dessen gesamte Laserstrahlaustrittsleistung für Laserwellenlängen jenseits des elektromagnetischen Spektrums ein Milliwatt übersteigt.

**Revendications**

1. Système thérapeutie au laser, tenu à la main et générant, un faisceau laser, comprenant un corps principal (1), contenant le générateur (1) de faisceau laser et terminé par une extrémité de sortie de forme conique qui à un alésage (6) se prolongeant le long de l'axe du corps et un capuchon (14) détachable, creux et de forme conique qui s'adapte sur l'extrémité de sortie de forme conique du corps principal (1) et est fixé à la têts par des moyens (15) pouvant s'enclencher avec des moyens (8) sur la tête, caractérisé en ce que l'orientation angulaire du capuchon est déterminée par des protubérances (17) dans le capuchon coulissant le long de guides (7) dans la tête et en ce que le capuchon comprend un tube (16) positionné le long de son axe intérieur central et soudé à l'extrémité du capuchon et qui est reçu par l'alésage (6) de façon à fournir un passage (5) pour le faisceau laser émis à travers le tube creux jusqu'à l'extérieur du capuchon.

2. Système suivant la revendication 1, caractérisé en ce que le capuchon (14) peut être fixé solidement à la tête par une nervure (15) sur la surface intérieure du capuchon, qui glisse dans une rainure (8) correspondante dans la tête.

3. Système suivant la revendication 2, caractérisé en ce qu'il y a, noyées dans le capuchon détachable, deux électrodes (9) qui établissent un contact avec des conduits respectifs (21) noyés dans la tête de l'unité laser, alors que le capuchon (14) est ajusté sur la tête si bien que la résistance de la peau localisée entre les deux extrémités (20) d'une sonde peut être déterminée, ce qui permet de déclencher l'émission du faisceau laser pour stimuler des points d'acupuncture.

4. Système suivant la revendication 2, caractérisé en ce que le capuchon détachable (14) a un prolongement (22) en forme de tige creuse à travers lequel le fasceau laser émis (5) se propage, bouché par un bloc solide (23) qui est transparent à la lumière laser et façonné de telle sorte que le faisceau laser qui se propage subit une réflexion interne totale à 90° pour sortie du capuchon par une surface convexe qui provoque la focalisation du faisceau laser sortant sur un petit point en face de la surface convexe (25), depuis lequel il diverge en un large faisceau conique (27) d'intensité décroissante par unité de surface.

5. Système suivant la revendication 4, caractérisé en ce que le tube de prolongement (22) en forme de tige creuse est bouché par un marquer de lignes consistant en un élément solide transparent (28) dont la surface d'entrée (29) est concave et dont la surface de sortie est convexe (31), le faisceau laser sortant (32) étant de section droite rectangulaire ou carrée fournissant une illumination de plus grande surface sur le corps du patient.

6. Système suivant la revendication 4, caractérisé en ce que la tige creuse (22) est bouchée par un solide transparent (33) dont l'extrémité a une cavité conique (35) pour provoquer une réflexion interne totale du faisceau laser transmis étalé sur 360° dans un plan à 90°, pour des traitements thérapeutiques d'organes internes dans le corps d'hommes et d'animaux.

7. Système suivant les revendications 5 et 6, caractérisé en ce que l'intensité du faisceau laser par unité de surface est inférieure à 1 milliwatt pour une aire circulaire de 7 mm de diamètre, mais dont la puissance totale d'émission du faisceau laser dépasse un milliwatt pour des longueurs d'onde laser dans le spectre électromagnétique.

FIG _ 1 _

FIG _ 2 _

FIG. 3

19

20

18  20

16

19

15

14

_FIG_ 4 _

_FIG. 5._

_FIG. 6._

_FIG. 7._

_FIG. 8._